# EUROPEAN PATENT SPECIFICATION

(11) **EP 1 827 899 B1**
(45) Date of publication and mention of the grant of the patent: **22.07.2009**
(21) Application number: 05853105.4
(22) Date of filing: 02.12.2005
(51) Int. Cl.: B60N 2/66

(54) **COMFORT BELT LUMBAR**
BEQUEMER RÜCKENGURT
SUPPORT LOMBAIRE A CEINTURE DE CONFORT

(30) Priority: 03.12.2004 US 632841 P; 02.06.2005 US 686579 P; 04.11.2005 US 733399 P
(43) Date of publication of application: 05.09.2007
(73) Proprietor: L&P PROPERTY MANAGEMENT COMPANY, South Gate, Ca 90280 (US)
(72) Inventor: COLJA, Renato, Windsor, Ontario N9G 2G7 (CA); KUKURUZOVIC, George, Windsor, Ontario N9E 4T7 (CA); MCMILLEN, Robert, J., Tecsumseh, Ontario N8N 2N4 (CA); JANZEN, Larry D., Harrow, Ontario N0R 1G0 (CA); NATANAIL, Adrian, V., Windsor, Ontario N9E 4T8 (CA); BAETENS, Eric, L., Amherstburg, Ontario N9V 4L1 (CA)
(74) Representative: Banzer, Hans-Jörg
(86) International application number: PCT/US2005/044094
(87) International publication number: WO 2006/060811

(56) References cited:
- US-A- 5 769 490
- US-A1- 2002 149 245
- US-A1- 2003 184 139
- US-A1- 2003 227 203

## Description

### CROSS-REFERENCE TO RELATED APPLICATIONS

This application claims priority to U.S. App. Nos. 60/632,841, 60/686,579 and 60/733,399, respectively filed on December 3, 2004, June 2, 2005 and November 4, 2005.

### BACKGROUND OF THE INVENTION

### FIELD OF THE INVENTION

The present invention relates to lumbar supports for seats, and more particularly to seats having a belt-type lumbar support.

### RELATED ART

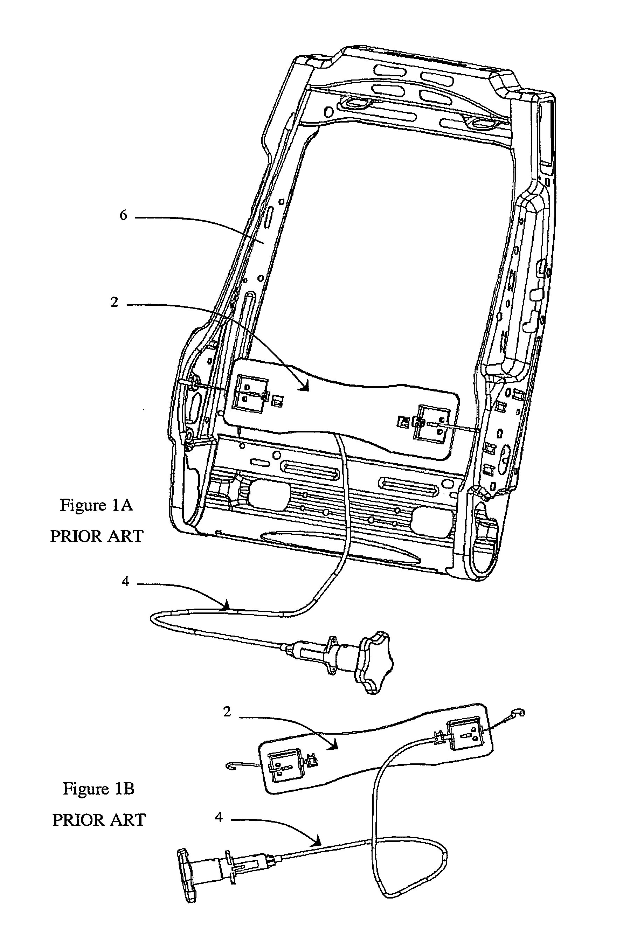

Many different types of strap lumbar supports have been used in seating systems. Strap or belt-type lumbar supports can be any type of strap, including a belt or wire, and are known to be supported in the seat frame by various means, including springs, hooks, brackets, clips, and wires. Strap lumbar supports are also known to be operated using a number of different actuation devices. An example of a prior art belt lumbar support is shown in Figures 1A and 1B of the accompanying drawings. Other examples of prior art strap lumbar devices can be found in the following references: US 3,258,259, US 3,273,877, US 4,155,592, US 4,309,058, US 4,462,635, US 5,224,757, US 5,482,353, US 5,507,559, US 5,685,606, US 5,716,098, US 5,769,490, US 5,788,328, US 5,797,652, US 5,860,700, US 6,152,531, US 6,254,186, US 6,394,546, US 6,412,868, US 6,471,294, US 6,644,740, US 6,918,634, US 2005/0023873, US 2004/0212227, EP 0296938, EP 0420824, EP 0540481, EP 0518830, EP 0582821, GB526572, FR 2596334, and FR 2765531.

Strap lumbar devices can usually be distinguished from arching lumbar devices, such as described and illustrated in US 6,003,941 and US 5,518,294, based on the difference between their respective tensioning elements. The strap lumbar is connected to the seat frame in a manner that the tensioning element must be pulled taut to increase support, and the tensioning element can be the strap itself, as with the belts in US 4,155,592, or another structure, as with the Bowden cable in EP 0296938, or a combination thereof as with support and cables in US 6,152,531. In comparison, the tensioning element in the arching lumbar is stiff and can be rotated, pushed or bowed to increase support. As particularly illustrated in Figure 1A and 1B, even in the case where the strap lumbar includes a rather stiff belt 2, the belt 2 is connected to a seat frame 6 through a traction cable 4. Increasing tension in the traction cable 4 pulls the belt 2 taut but does not arch the belt 2. As discussed below with reference to the present invention, the preferred traction cable 4 is a Bowden cable 50 which has a sheath 52 surrounding an inner wire core 54 that can slide within the sheath 52.

In belt lumbar devices, there are a number of different designs for the support elements and their cooperative relationship with the tensioning elements. Many strap lumbar supports are more flexible than the belt lumbar supports. Some supports form a part of the tensioning element and are themselves pulled taut between the seat frame, whereas other supports are merely pressed forward into the seat as the tensioning element is pulled taut.

Although many prior art devices have adequately provided lumbar support, some problems still remain. As a first example, the spring or cable of the strap-type lumbar support may wear, dig into, or catch upon the seat back foam causing the seat back foam to prematurely fail. As a second example, the belt-type lumbar support may twist or turn during installation or when in use by a seat occupant. The rotation or twisting of the belt-type lumbar support causes difficulty in installation and may cause discomfort to the seat occupant.

Modular seat assembly techniques require components to be designed for ease and speed of assembly. Modular lumbar supports often include vertical extensions. In both assembly and finished use, the extra leverage such extensions place on anchors can cause undesirable wear and binding. Accordingly, along with the need for strong, economical, compact components in seat design, there is a continuing need in seat assembly procedures to increase the economy, speed and efficiency of component assembly and shipping. Furthermore, as described in US Pub. No. 2004/0108760 and US 5,553,919, there is a benefit for providing spinal relief when supporting the spine or for using a shaped pad to provide comfort. It is preferable to apply lumbar supporting pressure bilaterally adjacent to the spinal column, while maintaining a vertical recess or channel to accommodate the vertebrae, because the bony vertebrae of the spine may become uncomfortable if direct pressure is applied to them by a lumbar support pressure surface.

Therefore, there is a need in the art for an improved belt-type lumbar support that does not affect the wear characteristics of the seat back foam. Further, there is a need in the art for an improved belt-type lumbar support that is rotationally fixed. Additionally, there continues to be a need for strong, economical, compact components that further increase the economy, speed and efficiency of component assembly and shipping. Finally, all of these structural and assembly criteria should be accomplished with the goal of maintaining the comfort of the seat.

### SUMMARY OF THE INVENTION

According to the present invention as defined in claim 1, a seat support includes a panel that is connected to opposite sides of the seat through a pair of fixtures and is operated by an actuator that is connected to at least one of the fixtures. The panel includes a recessed center channel that is bounded by planar side plates.
The panel may have side extensions at the ends of the side plates that connect the ends of the panel to the seat frame through a bracket. The extensions can include hinges, platforms and/or mounting hooks.

Further areas of applicability of the present invention will become apparent from the detailed description provided hereinafter. It should be understood that the scope of the invention is only limited by the appended claims.

### BRIEF DESCRIPTION OF THE DRAWINGS

The present invention will become more fully understood from the detailed description and the accompanying drawings, wherein:

Figures 1A and 1B are views of a prior art design for a belt lumbar.

Figures 2A and 2B are front and back, perspective views of the belt lumbar according to the present invention, respectively.

Figure 3A, 3B and 3C are back views of alternative embodiments of the belt lumbar according to the present invention.

Figures 4A, 4B and 4C are side views of alternative embodiments of the belt lumbar according to the present invention.

Figure 5 is a perspective view of an embodiment of the belt lumbar with side extensions as installed in a seat frame.

Figure 6 is a detailed view of the bracket illustrated in Figure 5.

Figures 7A and 7B are front and back views of the belt lumbar illustrated in Figure 5, respectively.

Figure 8 is an alternative embodiment of the belt lumbar with side extensions.

Figure 9 is another alternative embodiment of the belt lumbar with side extensions.

Figure 10 is a detail view of the belt lumbar attachment.

Figures 11 and 12 are detailed views of the belt lumbar extensions.

Figures 13-15 are perspective views of a similar belt lumbar panel with different installation configurations.

Figures 16 and 17 are perspective views of different belt lumbar combinations.

### DETAILED DESCRIPTION OF THE PREFERRED EMBODIMENTS

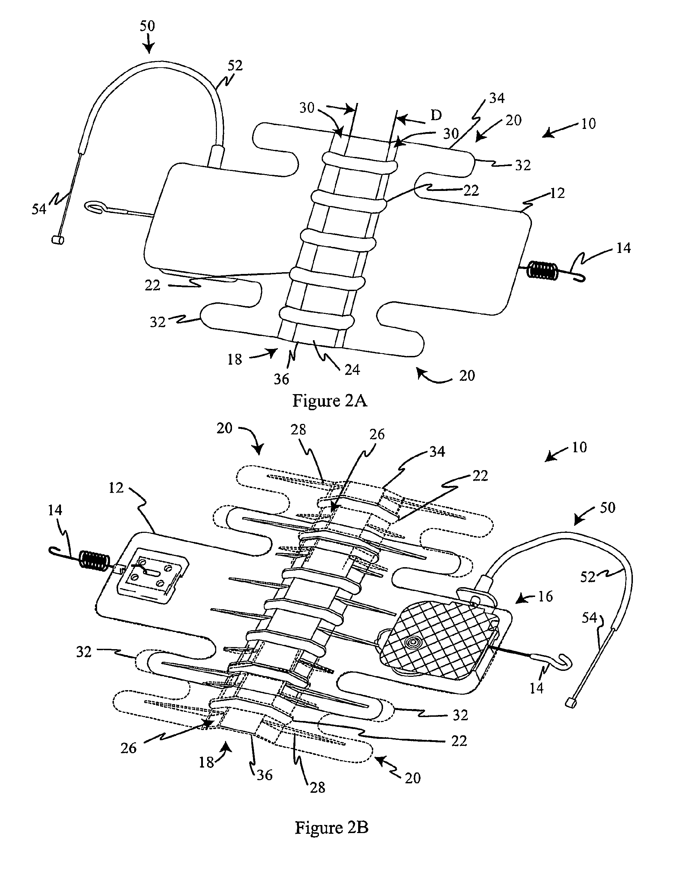

Referring to the accompanying drawings in which like reference numbers indicate like elements, Figures 2A and 2B illustrate perspective views of a belt lumbar 10 according to the present invention. The belt lumbar 10 includes a panel 12, a pair of fixtures 14, and an actuator 16 with a tensioning element connecting the panel 12 to a seat (not shown) through at least one of the fixtures 14. The panel is connected to opposite sides of the seat through the fixtures 14 and can be pulled taut in the seat by the actuator or the tensioning element of the actuator may be pulled taut to force the panel 12 forward into the seat.

The panel 12 includes a recessed center channel 18 that is bounded by planar side plates 20 and may include apertures 22 extending across the channel. The recessed center channel 18 has a bottom trough 24 between a pair of sloping, faceted sides 26, and can be formed by concave ribs 30 that extend between the pair of side plates 20, i.e. extending transverse to the recessed center channel 18 and separated by the apertures 22. As particularly illustrated in Figure 2, the side plates 20 are preferably formed with ridges 28 that are in line with and connect to the concave ribs 30. The ridges 28 extend transversely to the channel 18.

The faceted sides 26 of the recessed center channel 18 are separated by a distance D. In the depicted embodiment, the faceted sides 26 are preferably separated by at least 30mm. The side plates 20 extend transversely on either side of the recessed center channel 18 in an elongated manner, i.e. the side plates 20 are more than twice as wide in comparison to the recessed center channel 18 or more than twice as wide as the distance D.

The size of the ridges 28 can be changed, increased or decreased, to vary the stiffness of the panel 12 in the direction transverse to the channel 18. The size of the apertures 22, i.e. width and spacing of the ribs 30, can also be varied to alter the stiffness of the panel 12 along the axis of the channel 18. It will also be appreciated that the number and size of the finger extensions 32 can also be varied to change the size of the support region. Generally, the side panels are more stiff or rigid in comparison with the channel 18.

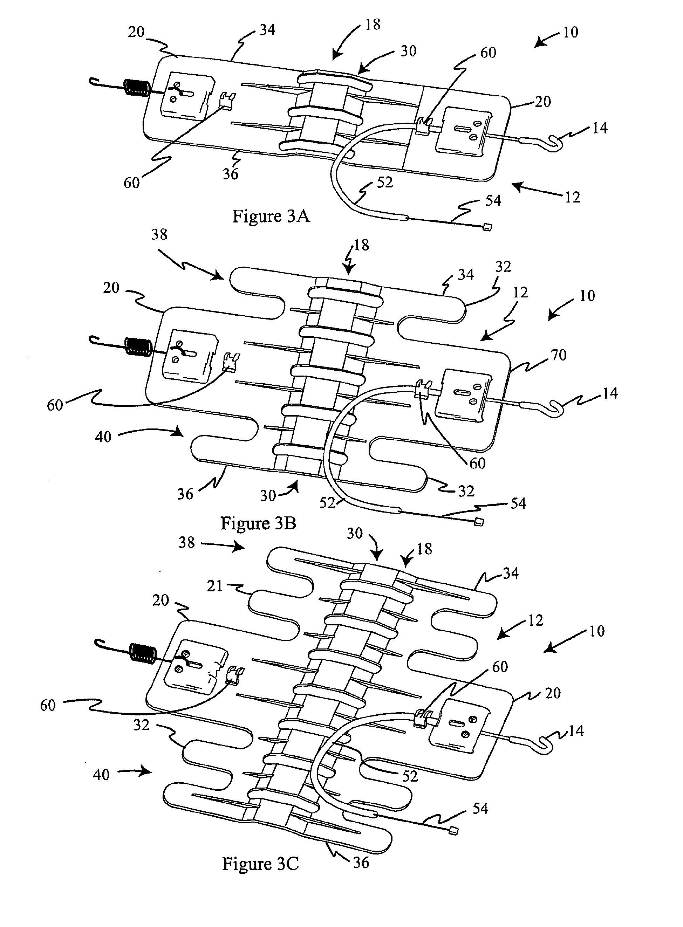

The recessed center channel 18 extends entirely along the length of the panel's centerline, from a top edge 34 to a bottom edge 36 shown in each of the preferred embodiments, as particularly illustrated in Figures 3A, 3B and 3C. In Figure 3A, the pair of side plates 20 and top and bottom ribs 30 form a panel 12 with straight top and bottom edges 34, 36. As illustrated in Figures 3B and 3C, the panel 12 could also have a top extension 38 and a bottom extension 40. In each of these embodiments, either one or both of these top and bottom extensions 38, 40 may also have side plates 20. These side plates 20 may also have flexible projections (or fingers) 32 extending transversely to the recessed channel 18 and in line with the concave ribs 30.

It will be appreciated that the panel 12 can be attached to a seat frame (not shown) through springs, hooks, brackets, clips, wires, and any other equivalent fastener or connector hardware. In the preferred embodiment, the tensioning element of the actuator 16 is a Bowden cable 50 which has a sheath 52 surrounding an inner wire core 54 that can slide within the sheath 52. The actuator 16 can be operated by a manual device, such as a hand wheel or lever, or by a powered device, such as an electric motor and gear assembly. The sheath 52 is attached to the panel 12 by a clip 60 and the wire core 54 extends to the connector 14.

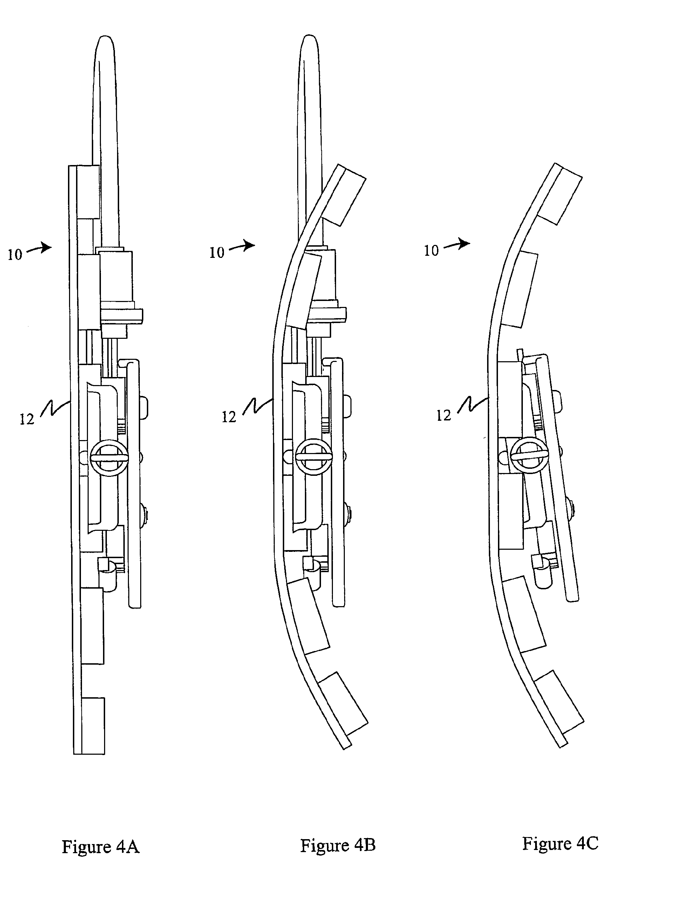

As illustrated in Figures 4A, 4B and 4C, the panel 12 can be formed with different support profiles. The panel 12 in Figure 4A is relatively flat, whereas the panels 12 in Figures 4B and 4C are arcuate, having an arc and a three-fold curve. Each of the panels in Figures 4A, 4B and 4C are similar in that they are substantially planar as they extend transversely from the recessed center channel 18.

From the prior art devices, it will be appreciated that there are a number of ways to connect the panel 12 to the seat frame 6 and provide tension thereto by one or more actuators 16. For example, as with US 6,152,531, the panel may be mounted to the frame through springs or other brackets and may have a tensioning element on each side of the panel. Alternatively, the Bowden cable may extend behind the panel from one side to the other, such as in EP 0296938. For the embodiments described above, it will be particularly noted that there is a clip 60 on each side of the panel 12 which can accommodate the sheath 52 for a Bowden cable that extends behind the panel 12 or for an actuator 16 on each side of the panel 12. As discussed below, there are additional ways to connect the panel 12 to the seat frame 6 that differ from known devices.

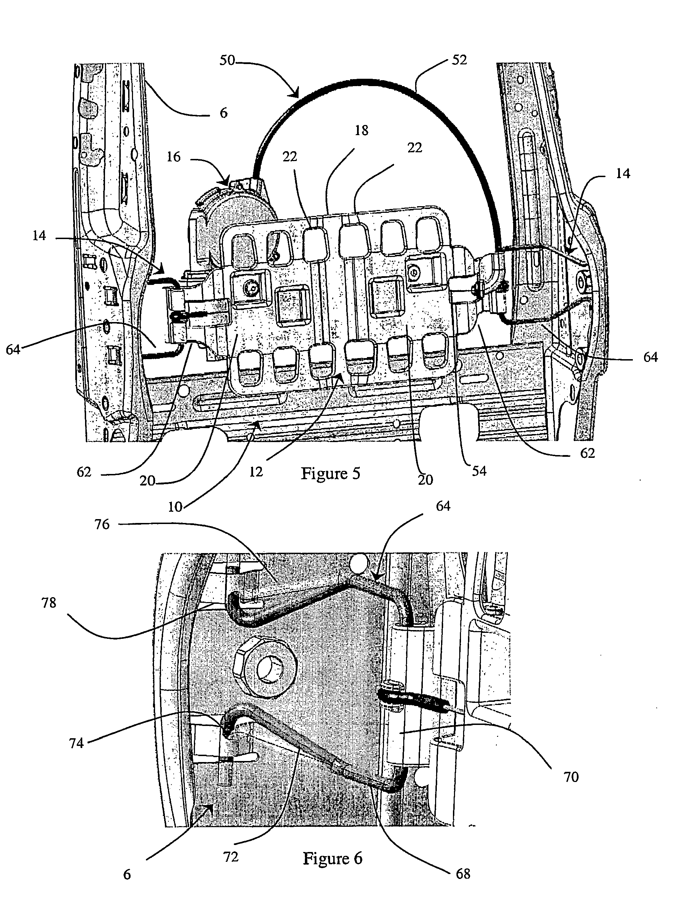

As with the panels 12 described in detail above with reference to Figures 2-4, the embodiments described below also have panels 12 formed with a recessed center channel 18. However, the embodiments in Figures 2-4 do not have any side extensions which would, during assembly, prevent the panel from rotating within the seat frame or maintain the panel in a fixed position within the seat frame. In comparison, the embodiments described below with reference to Figures 5-9 have side extensions 62 that maintain the panel 12 within the seat frame 6 and restrain the panel 12 against rotation.

As illustrated in Figure 5, the belt support 10 can be connected to the seat frame 6 through a pair of brackets 64 at opposite sides of the belt support 10, and a Bowden cable 50 that is also connected between the brackets 64. The belt support 10 includes the panel 12 and the pair of side extensions 62 that respectively extend between the panel 12 and the brackets 64 which hold the lumbar support 10 within the seat frame. The side extensions 62 are preferably in the form of hinged extensions 66 that are discussed in more detail below. As discussed above, the panel 12 includes a center recessed channel 18 between a pair of side plates 20, and the hinged extensions 66 and the brackets 64 restrict said panel 12 from rotating.

As particularly illustrated in Figure 6, the brackets 64 are preferably formed by a u-shaped support wire 68. The hinged extensions 66 connect to the brackets 64 at the center portion 70 of the u-shaped wire. A leg portion 72 with a flared end 74 is at each end of the center portion 70. The legs 72 can flex 76 to permit the flared ends 74 to fit into lance tabs 78 in the seat frame 6. The center portion 70 may be angled and can provide flexibility to the bracket 64 in a spring-like manner.

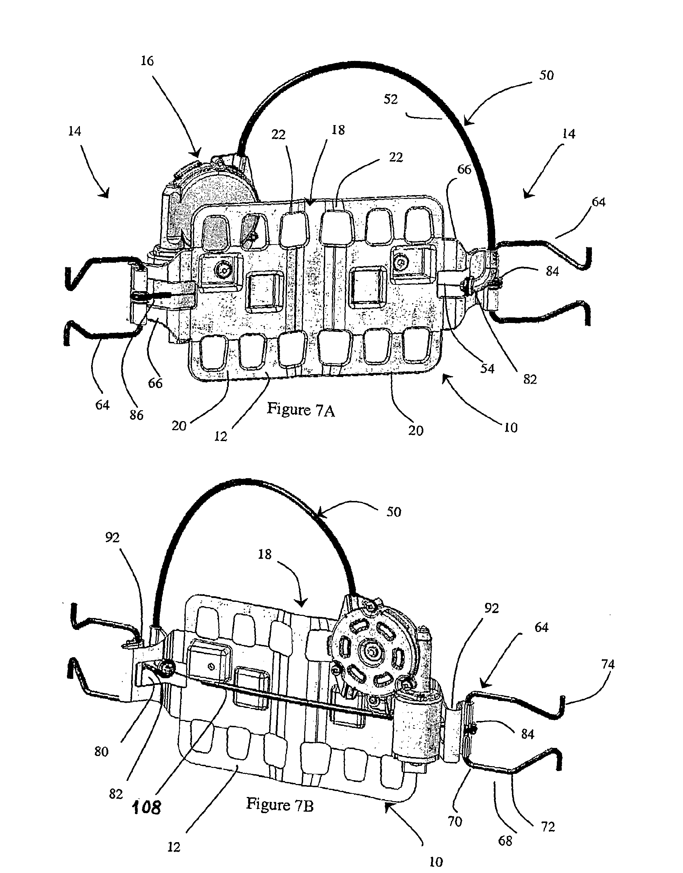

Figures 7A and 7B demonstrate the modular nature of the lumbar support 10 for assembly operations. The actuator 16 is directly attached to the panel 12 and the brackets 64 are fitted into the ends of the side extensions 62, with the Bowden cable 50 extending therebetween. With this configuration, the entire lumbar support 10 can be installed as a unit within the seat frame 6 by merely fitting the flared ends 74 into the lance tabs 78 and connecting the actuator 16 to its source for power and control. Once installed, as the wire 54 in the Bowden cable 50 is pulled through the sheath 52, the tension in the Bowden cable 50 increases and as it becomes taut, thereby forcing the panel 12 forward in the seat frame 6.

The hinged extensions 66 preferably include a central aperture 80 through which the Bowden cable 50 extends and fittings for the Bowden cable sheath 52 and cable 54. In particular, at the end of one of the side extensions 62, there is a mount 82 which holds the end of the sheath 52 in place. The mount 82 is preferably held in place on the side extension by a snap-fit connection 84, which can also be an indentation or a groove for a form fit connection. Similarly, at the end of the other side extension 62, a snap fit connection 84 is preferably used as the fitting for a hook 86 at the end of the wire 54. The sheath 52 that extends from the actuator 16 ends at the mount 82, but as particularly illustrated in Figure 7B, a sheath segment 108 may also extend between the brackets.

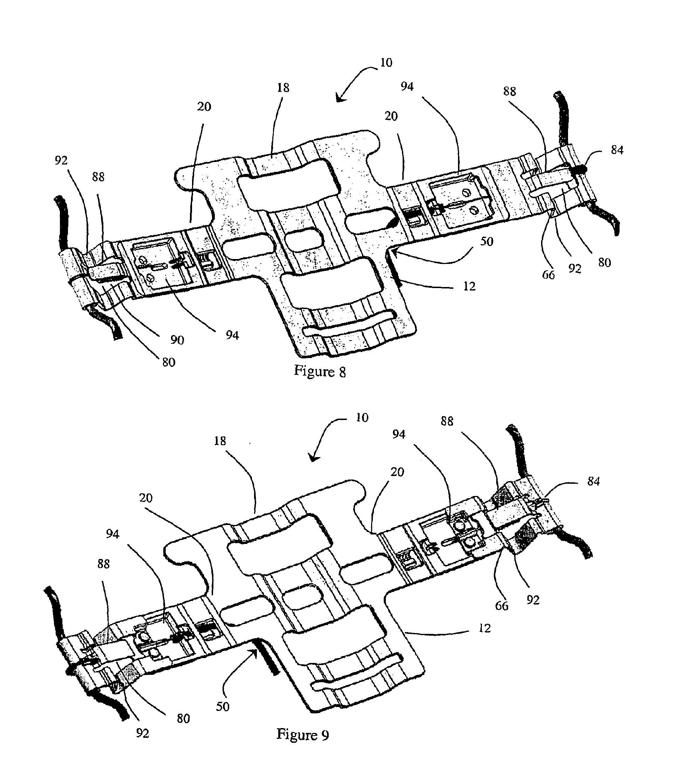

Figures 8 and 9 illustrate alternative attachment configurations for hinged extensions 66 with an optional tab 88 in front of the aperture 80. In these configurations, the sheath 52 is connected to the side plate 20 on one side of the recessed center channel 18, with the wire 54 extending behind the tab 88 and through the aperture 80, and a spring 90 is connected to the other side plate 20 on the other side of the recessed center channel 18. Similar to the wire 54, the spring 90 extends through the aperture 80 and connects to the ends of the side extensions 62. With the tab 88 in front of the aperture 80, the padding at the front of the seat (not shown) is less likely to get caught, worn or torn by the hinged extensions 66, the spring 90 or the wire 54 as the panel 12 moves forward in the seat frame and pushes against the padding and the hinged extensions 66 close.

The hinged extensions 66 can be integrally formed with the panel 12, as in Figure 8, or fastened to the side plates 20, as in Figure 9. Also, the tab 88 can extend over the aperture 80 outwardly from the side plates 20 or inwardly from the ends of the hinged extensions 66. In the prior art, U.S. Patent No. 5,716,098 discloses a flexible hinge with a tongue and slot arrangement. However, the tongue and slot are in a different orientation and relative relationship with respect to the belt and each other. In particular, the flexible hinge in the prior art is not formed as a hinge extension to the belt and does not have an aperture. In comparison, U.S. Patent No. 5,769,490 discloses another type of a flexible hinge which can distort in an elastic manner. This type of flexible hinge is not formed as an extension at the end of the belt but is instead formed between the ends of the belt and also does not have an aperture as in the present invention. Even though the hinge extensions 66 of the present invention preferably have a pre-formed curvature 92 to provide more area for the aperture 80, it is possible that each end of the belt lumbar 10 could use a pair of elastic-type flexible hinges without any such pre-formed curvature such that an aperture is formed between the flexible hinges. It is also possible for a living hinge to be used for the hinge extension 66, in which case the angles in the pre-formed curvature 92 would preferably be thinner in cross-section than the side plates 20. As discussed above, the hinge extension 66 permits the panel 12 to move toward or away from a seat occupant but significantly reduces the ability of the panel 12 to twist or rotate between the frame 6. Therefore, in comparison with these prior art references, by using the hinge extensions 66, the present invention serves to reduce any rotation or twisting of the panel 12 between the frame 6, whereas the single flexible hinge designs are designed to permit the rotation or twisting of the belt lumbar.

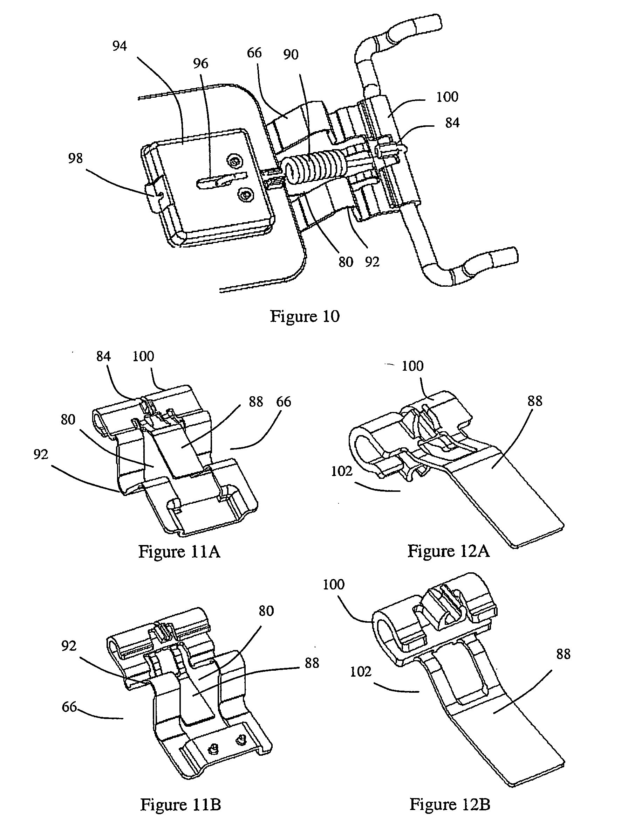

As generally illustrated in Figures 8 and 9 and particularly illustrated in Figure 10, the side plates 20 can also contain a pocket 94. The pocket 94 includes a slot 96 to receive an end of the spring 90. The pocket 94 may also be reinforced, such as with additional material in this region or by an insert 98 of plastic or metal. As also illustrated in Figure 10, the hinged extensions 66 are preferably formed with a clip 100 at the end to secure the panel 12 to the brackets 64. Preferably, the clip 100 has a hooked end that fits snugly over the center portion 70 of the u-shaped support wire 68.

Figures 11 and 12 compare the differences between the preferred hinge extension 66 that connects the panel 12 to the bracket to prevent rotation or twisting and an straight extension 102 that permits the panel to rotate and twist. Figures 11A and 11B respectively show the front and back views of the hinge extension 66, and Figures 12A and 12B respectively show the front and back views of the straight extension 102. Each type of extension 66, 102 includes the tab 88 in front of the aperture 80 to prevent the padding from getting caught, worn or torn by the spring 90 or the wire 54 as the panel 12 moves forward in the seat frame. Additionally, each of these extensions 66, 102 includes a clip 100 and snap-fit connections 84. However, the straight extension 102 does not connect the panel to the bracket. Instead, the panel 12 is only connected to the brackets through the Bowden cable. Of course, as with the lumbar system disclosed by EP 0296938, additional springs or brackets could connect the panel to the seat frame apart from the tension cable.

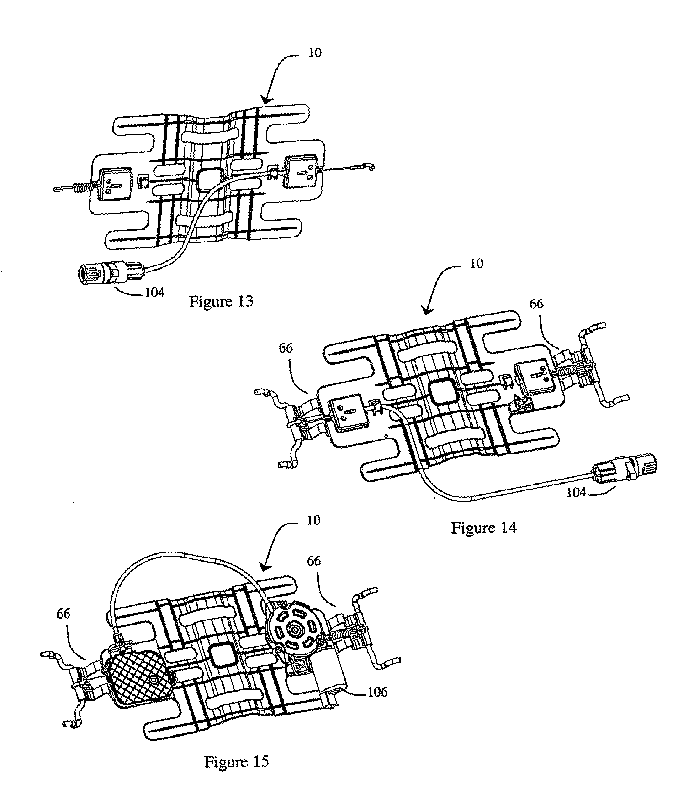

Figures 13-15 each show a belt lumbar 10 with different installation configurations. Each one of the installations uses a panel 12 similar to the one described above with reference to Figure 3B. In Figure 13, the belt lumbar 10 has a panel 12 without any extensions and a manually-operated actuator 104 is used to provide tension to the Bowden cable. In Figure 14, the belt lumbar 10 has hinged extensions 66 connecting the ends of the panel 12 with the brackets, and this configuration also has a manually-operated actuator 104. In Figure 15, the belt lumbar 10 has hinged extensions 66 and has a power-operated actuator 106.

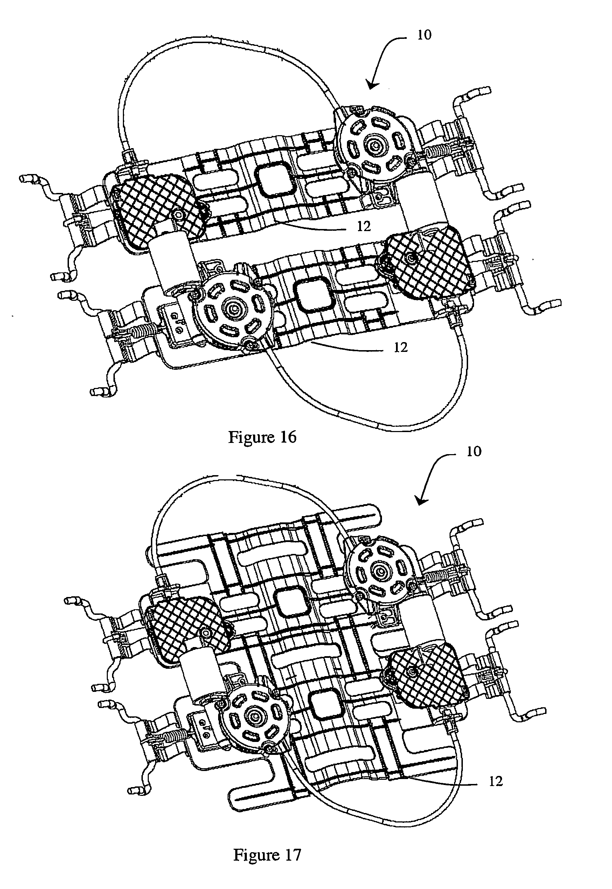

Figures 16 and 17 are perspective views of different belt lumbar combinations according to the present invention. In Figure 16, there are two panels 12 that are spaced vertically with respect to each other, and each has its own actuator 16. In Figure 17, there is a single panel 16 that has vertically-spaced actuators. The single panel may actually be a pair of panels that are vertically adjacent to each other.

As various modifications could be made to the exemplary embodiments, as described above with reference to the corresponding illustrations, without departing from the scope of the invention, it is intended that all matter contained in the foregoing description and shown in the accompanying drawings shall be interpreted as illustrative rather than limiting. Thus, the breadth and scope of the present invention should not be limited by any of the above-described exemplary embodiments, but should be defined only in accordance with the following claims appended hereto.

## Claims

1. A lumbar support for a seat, comprising:
a belt support comprising a panel (12) having a recessed center channel (18) bounded by a pair of side plates (20), wherein said panel (12) further comprises a top edge (34) and a bottom edge (36) and said recessed center channel (18) extends entirely from said top edge (34) to said bottom edge (36), and wherein said side plates (20) transversely extend from said center channel in an elongated manner, said recessed center channel (18) facing toward a seat occupant;
a pair of fixtures (14) attached to said side plates and connecting said panel to opposite sides of the seat in a suspended manner; and
an actuator (16) operatively connected to at least one of said pair of fixtures (14).

2. A lumbar support according to claim 1, wherein said pair of fixtures are comprised of first and second brackets (64), each comprised of a support wire (68) having a center portion (70) between a pair of flexible legs (72) with flared ends (74), and wherein said panel (12) is further comprised of a pair of hinged extensions (66) respectively extending between said side plates (20) and said first and second brackets (64).

3. A lumbar support according to claim 2, wherein said hinged extensions (66) are further comprised of a snap-fit connection (84).

4. A lumbar support according to any one of claims 1-3 wherein said recessed center channel (18) comprises a plurality of apertures (22).

5. A lumbar support according to any one of claims 1-4, wherein said recessed center channel (18) further comprises a bottom trough (24) between a pair of sloping, faceted sides (26).

6. A lumbar support according to claim 5, wherein said apertures (22) form a plurality of concave ribs (30) extending between said pair of side plates (20) transverse to said recessed center channel (18).

7. A lumbar support according to claim 6, wherein each of said pair of side plates (20) further comprises a plurality of ridges (28) extending transversely to said channel (18), wherein said ridges (28) respectively are in line with and connect to said concave ribs (30).

8. A lumbar support according to any one of claims 1-7, wherein said panel (12) further comprises a top extension (38) and a bottom extension (40), each of which comprises a recessed channel extension bounded by a pair of side extensions (20).

9. A lumbar support according to any one of claims 1-8, wherein said fixtures (14) further comprises any one selected from the group of connectors consisting of springs, hooks, clips, wires, and any combination thereof.

10. A lumbar support according to any one of claims 1-9, wherein said actuator is comprised of a bowden cable (50).

11. A lumbar support according to claim 2,
wherein at least one of said pair of hinged extensions (66) comprises an aperture (80); wherein said actuator further comprises a bowden cable (50) comprising an outer sheath (52) and an inner wire (54), wherein at least one of said outer sheath (52) and said inner wire (54) extends through said aperture (80) and is connected to one of said first and second brackets (64); and
said lumbar support further comprising a spring (90) extending between said panel (12) and said first bracket (64) through said aperture (80).

12. A lumbar support according to claim 11, wherein said outer sheath (52) is connected to said panel (12) by a clip and said inner wire (54) is connected to said second bracket (64).

13. A lumbar support according to claim 11 or 12, wherein at least one of said hinged extensions (66) further comprises a tab (88) in front of said spring (90) as it extends through said aperture (80).

## Patentansprüche

1. Lordosenstütze für einen Sitz, umfassend:
eine Gurtstütze umfassend eine Platte (12) mit einem ausgesparten mittleren Kanal (18), welcher von einem Paar von Seitenplatten (20) begrenzt ist, wobei die Platte (12) ferner einen oberen Rand (34) und einen unteren Rand (36) umfasst, und wobei sich der ausgesparte mittlere Kanal (18) gänzlich von dem oberen Rand (34) zu dem unteren Rand (36) erstreckt, und wobei sich die Seitenplatten (20) quer verlaufend von dem mittleren Kanal in einer verlängerten Art und Weise erstrecken, wobei der ausgesparte mittlere Kanal (18) einem Sitzinsassen zugewandt ist;
ein Paar von Befestigungen (14), welche an den Seitenplatten angebracht sind und die Platte an gegenüberliegenden Seiten des Sitzes in einer gespannten Art und Weise befestigen; und
eine Betätigungsvorrichtung (16), welche funktionsfähig mit mindestens einer von dem Paar von Befestigungen (14) verbunden ist.

2. Lordosenstütze nach Anspruch 1, wobei das Paar von Befestigungen eine erste und eine zweite Halterung (64) umfasst, die jeweils einen Stützdraht (68) mit einem mittleren Abschnitt (70) zwischen einem Paar von flexiblen Beinen (72) mit aufgeweiteten Enden (74) umfassen, und wobei die Platte (12) ferner ein Paar von schwenkbaren Erweiterungen (66) umfasst, welche sich jeweils zwischen den Seitenplatten (20) und der ersten und der zweiten Halterung (64) erstrecken.

3. Lordosenstütze nach Anspruch 2, wobei die schwenkbaren Erweiterungen (66) ferner eine Schnappverschlussverbindung (84) umfassen.

4. Lordosenstütze nach einem der Ansprüche 1-3, wobei der ausgesparte mittlere Kanal (18) mehrere Öffnungen (22) umfasst.

5. Lordosenstütze nach einem der Ansprüche 1-4, wobei der ausgesparte mittlere Kanal (18) ferner eine Bodenwanne (24) zwischen einem Paar von schrägen, facettierten Seiten (26) umfasst.

6. Lordosenstütze nach Anspruch 5, wobei die Öffnungen (22) mehrere konkave Rippen (30) ausbilden, welche sich zwischen dem Paar von Seitenplatten (20) quer zu dem ausgesparten mittleren Kanal (18) erstrecken.

7. Lordosenstütze nach Anspruch 6, wobei jede von dem Paar von Seitenplatten (20) ferner mehrere Erhöhungen (28) umfasst, welche sich quer zu dem Kanal (18) erstrecken, wobei die Erhöhungen (28) jeweils ausgerichtet zu und verbunden mit den konkaven Rippen (30) sind.

8. Lordosenstütze nach einem der Ansprüche 1-7, wobei die Platte (12) ferner eine Oberseitenerweiterung (38) und eine Unterseitenerweiterung (40) umfasst, welche jeweils eine ausgesparte Kanalerweiterung umfassen, welche von einem Paar von Seitenerweiterungen (20) begrenzt wird.

9. Lordosenstütze nach einem der Ansprüche 1-8, wobei die Befestigungen (14) ferner eine beliebige ausgewählte Befestigung aus der Gruppe bestehend aus Federn, Haken, Klipps, Drähten und eine beliebige Kombination daraus umfassen.

10. Lordosenstütze nach einem der Ansprüche 1-9, wobei die Betätigungsvorrichtung einen Bowdenzug (50) umfasst.

11. Lordosenstütze nach Anspruch 2,
wobei mindestens ein Paar von schwenkbaren Erweiterungen (66) eine Öffnung (80) umfasst;
wobei die Betätigungsvorrichtung ferner einen Bowdenzug (50) umfasst, welcher eine äußere Hülle (52) und einen inneren Draht (54) umfasst, wobei sich die äußere Hülle (52) und/oder der innere Draht (54) durch die Öffnung (80) erstreckt und mit entweder der ersten oder der zweiten Halterung (64) verbunden ist; und
wobei die Lordosenstütze ferner eine Feder (90) umfasst, welche sich zwischen der Platte (12) und der ersten Halterung (64) durch die Öffnung (80) erstreckt.

12. Lordosenstütze nach Anspruch 11, wobei die äußere Hülle (52) mit der Platte (12) über einen Klipp verbunden ist und der innere Draht (54) mit der zweiten Halterung (64) verbunden ist.

13. Lordosenstütze nach Anspruch 11 oder 12, wobei mindestens eine der schwenkbaren Erweiterungen (66) ferner eine Zunge (88) vor der Feder (90) umfasst, während die sich durch die Öffnung (80) erstreckt.

## Revendications

1. Support lombaire pour un siège, comprenant :
un support de ceinture comprenant un panneau (12) comportant un canal central en retrait (18) limité par une paire de plaques latérales (20), ledit panneau (12) comprenant en outre un bord supérieur (34) et un bord inférieur (36) et ledit canal central en retrait (18) s'étendant entièrement dudit bord supérieur (34) jusqu'audit bord inférieur (36), et lesdites plaques latérales (20) s'étendant transversalement dudit canal central d'une manière allongée, ledit canal central en retrait (18) faisant face à un occupant de siège ;
une paire de fixations (14) fixées auxdites plaques latérales et reliant d'une manière suspendue ledit panneau à des côtés opposés du siège ; et
un actionneur (16) relié fonctionnellement à au moins l'une de ladite paire de fixations (14).

2. Support lombaire selon la revendication 1, dans lequel ladite paire de fixations est composée de premier et deuxième supports (64), composés chacun d'un fil de support (68) ayant une partie centrale (70) entre une paire de pattes (72) flexibles avec des extrémités évasées (74), et dans lequel ledit panneau (12) est en outre composé d'une paire d'extensions articulées (66) s'étendant respectivement entre lesdites plaques latérales (20) et lesdits premier et deuxième supports (64).

3. Support lombaire selon la revendication 2, dans lequel lesdites extensions articulées (66) sont en outre composées d'une liaison par encliquetage (84).

4. Support lombaire selon l'une quelconque des revendications 1 à 3, dans lequel ledit canal central en retrait (18) comprend une pluralité d'ouvertures (22).

5. Support lombaire selon l'une quelconque des revendications 1 à 4, dans lequel ledit canal central en retrait (18) comprend en outre un creux inférieur (24) entre une paire de côtés (26) inclinés à facettes.

6. Support lombaire selon la revendication 5, dans lequel lesdites ouvertures (22) forment une pluralité de nervures concaves (30) s'étendant entre ladite paire de plaques latérales (20) transversalement audit canal central en retrait (18).

7. Support lombaire selon la revendication 6, dans lequel chacune de ladite paire de plaques latérales (20) comprend en outre une pluralité d'arêtes (28) s'étendant transversalement audit canal (18), lesdites arêtes (28) étant respectivement alignées avec lesdites nervures concaves (30) et reliées à celles-ci.

8. Support lombaire selon l'une quelconque des revendications 1 à 7, dans lequel ledit panneau (12) comprend en outre une extension supérieure (38) et une extension inférieure (40), chacune comprenant une extension de canal en retrait limitée par une paire d'extensions latérales (20).

9. Support lombaire selon l'une quelconque des revendications 1 à 8, dans lequel lesdites fixations (14) comprennent en outre un raccord sélectionné dans le groupe de raccords consistant en des ressorts, des crochets, des agrafes, des fils, et n'importe quelle combinaison de ceux-ci.

10. Support lombaire selon l'une quelconque des revendications 1 à 9, dans lequel ledit actionneur est composé d'un câble bowden (50).

11. Support lombaire selon la revendication 2,
dans lequel au moins l'une de ladite paire d'extensions articulées (66) comprend une ouverture (80) ;
dans lequel ledit actionneur comprend en outre un câble bowden (50) comprenant une gaine externe (52) et un fil interne (54), au moins l'un de ladite gaine externe (52) et dudit fil interne (54) s'étendant à travers ladite ouverture (80) et est relié à l'un desdits premier et deuxième supports (64) ; et
ledit support lombaire comprenant en outre un ressort (90) s'étendant entre ledit panneau (12) et ledit premier support (64) à travers ladite ouverture (80).

12. Support lombaire selon la revendication 11, dans lequel ladite gaine externe (52) est reliée audit panneau (12) par une agrafe et ledit fil interne (54) est relié audit deuxième support (64).

13. Support lombaire selon la revendication 11 ou 12, dans lequel au moins l'une desdites extensions articulées (66) comprend en outre une languette (88) face audit ressort (90) alors qu'il s'étend à travers ladite ouverture (80).
